# FASCICULE DE BREVET EUROPEEN

(11) **EP 1 596 729 B1**
(45) Date de publication et mention de la délivrance du brevet: **06.04.2011**
(21) Numéro de dépôt: 04713568.6
(22) Date de dépôt: 23.02.2004
(51) Int. Cl.: A61B 17/122, A61B 17/128, A61B 17/08

(54) **DISPOSITIF POUR LIGATURER UNE STRUCTURE ANATOMIQUE**
VORRICHTUNG ZUM ABBINDEN VON ANATOMISCHEN STRUKTUR
DEVICE FOR LIGATURING AN ANATOMICAL STRUCTURE

(30) Priorité: 24.02.2003 FR 0302216
(43) Date de publication de la demande: 23.11.2005
(73) Titulaire: SOFRADIM PRODUCTION, 01600 Trévoux (FR)
(72) Inventeur: SGRO, Jean-Claude, F-21000 DIJON (FR)
(74) Mandataire: Cardy, Sophie Marie
(86) Numéro de dépôt international: PCT/FR2004/000403
(87) Numéro de publication internationale: WO 2004/075762

(56) Documents cités:
- EP-A- 0 637 432
- WO-A-01/34038
- DE-A- 1 957 855
- US-A- 4 592 355
- US-A- 5 609 599
- US-A- 5 749 879
- US-A- 5 766 189
- US-A- 5 782 839
- US-B1- 6 261 303

## Description

L'invention concerne un dispositif pour ligaturer une structure anatomique.

Le dispositif selon l'invention s'inscrit dans le cadre des matériels chirurgicaux permettant de réaliser des actes chirurgicaux notamment dans les espaces réduits en particulier sous coelioscopie. Cette technique mini-invasive comportant l'utilisation d'instruments fins, cylindriques, passés à travers la paroi abdominale sans utiliser de grande ouverture rend impossible l'utilisation des doigts ou de la main.

En ce qui concerne, par exemple, la résection chirurgicale de l'appendice iléo-cæcal, l'intervention est relativement simple lorsqu'elle est réalisée par voie ouverte conventionnelle ; il faut contrôler puis couper les vaisseaux qui alimentent l'appendice et qui sont inclus dans un feuillet anatomique nommé méso-appendice. Puis il faut lier la jonction de la base de l'appendice et du caecum, partie inférieure du colon droit. Enfin, il faut couper l'appendice juste au-dessus de la ligature. La technique coelloscopique reprend ces temps opératoires, mais représente une technique plus difficile. La difficulté posée par la section du méso-appendice est résolue actuellement pour cette voie, soit par l'utilisation du bistouri électrique, soit par des clips métalliques. Mais l'utilisation d'énergie électrique, dangereuse, est à éviter. Quant à l'utilisation de clips métalliques, cela demande un matériel complexe, coûteux, parfois de taille inadaptée. La ligature de la base appendiculaire, a priori évidente, est souvent beaucoup plus difficile que prévue. Des noeuds coulants pré-montés sont disponibles mais ils sont souples, leur passage autour de l'appendice demande beaucoup d'attention, leur serrage doit être fait avec précaution, pour ne pas couper complètement l'appendice, mais il doit suffisamment être fort pour que le noeud ne se défasse pas et que les tissus ne glissent pas.

L'état de la technique est illustré par WO 01/34038, US 5,476,206 et US 5,433,721.

WO 01/34038 décrit un appareil permettant de saisir le moignon appendiculaire et de faire glisser sur lui un lien élastique. On procède de même pour le méso-appendice. Cela suppose que l'appendice soit assez mince et souple pour être inclus dans l'appareil, de même pour le méso-appendice, ou que la base de l'appendice et le méso aient déjà été coupés.

Dans certains cas particuliers où l'appendice est extrêmement remanié ou a augmenté de volume, il peut être nécessaire d'utiliser des appareillages plus complexes et coûteux, tels que ceux décrits dans les brevets US 5,476,206 et US 5,433,721. Ces appareillages permettent de poser deux rangées d'agrafes et de couper entre les deux rangées. Le résultat est certes satisfaisant mais on ne peut pas utiliser systématiquement ces appareillages car ils nécessitent des accès plus gros que ceux habituellement réalisés pour la résection chirurgicale de l'appendice et ils coûtent chers. Les accès nécessaires avec ces appareils ont en effet un diamètre compris entre 10 et 12 mm au lieu de 5 mm.

Des dispositifs individuels à usage unique peuvent convenir, car deux attaches peuvent suffire pour le traitement de lésions simples, d'une appendicectomie, ou de ligature isolée de vaisseaux sanguins.

L'invention concerne donc un dispositif pour ligaturer une structure anatomique oblongue, telle qu'un appendice iléo-cæcal, un méso-appendice ou un vaisseau sanguin, comportant un clip et un applicateur, formant un tout qui ne se dissocie qu'à la fin de la ligature, ce dispositif devant être manipulé d'une seule main pour crocheter la structure anatomique, la lier et éventuellement la couper.

Le document US 5 609 599 porte sur un dispositif qui comprend d'une part, un élément de ligature comportant une première branche et une deuxième branche présentant chacune une extrémité proximale une extrémité distale une face interne et une face externe, une bande mince flexible formant charnière et reliant les extrémités distales desdites deux branches et des moyens pour lier entre elles au moins les portions proximales desdites deux branches, lesdites deux branches ménageant entre elles, après leur liaison, un canal transversal enserrant une portion de ladite structure anatomique, et
d'autre part, un applicateur pour mettre en place ledit élément de ligature, ledit applicateur comportant :
- un corps cylindrique ayant une extrémité distale et une extrémité proximale
- un logement pour recevoir ledit élément de ligature , ce logement présentant une paroi latérale prévue à l'extrémité distale dudit corps et contre laquelle la face externe de la première branche dudit élément de ligature est en appui notamment lors de son transport,
- des moyens pour écarter la deuxième branche de la première branche afin de permettre le crochetage de la structure anatomique à ligaturer, et
- des moyens pour rapprocher la deuxième branche de la première branche afin de lier entre elles lesdites deux branches.

Il n'existe pas jusqu'à présent d'autres matériels permettant de réaliser les différents temps chirurgicaux.

Le but de l'invention est de pallier à ce manque.

Selon l'invention, ce dispositif est caractérisé par le fait qu'il comprend en outre un coulisseau permettant d'agir sur les moyens pour écarter ou rapprocher la deuxième branche de la première branche.

Selon une caractéristique avantageuse le coulisseau est monté mobile au moins le long de la paroi latérale dudit logement, ledit coulisseau comportant les moyens pour écarter ou rapprocher la deuxième branche de la première branche et étant susceptible d'être déplacé axialement par un organe de manoeuvre disposé à l'extrémité proximale dudit corps et relié audit coulisseau par une tige montée coulissante dans ledit corps.

Selon un premier mode de réalisation de l'élément de ligature selon l'invention, les deux branches sont rectilignes et rigides et les moyens pour lier entre elles lesdites deux branches comportent des éléments de clippage mâles et des éléments de clippage femelles prévus sur les faces internes desdites deux branches.

Avantageusement les éléments de clippage femelles comportent au moins une gorge longitudinale à contre-dépouille débouchant dans la face interne de l'une des branches, et les éléments de clippage mâles comportent au moins une paroi cylindrique formée sur la face interne de l'autre branche et susceptible d'être introduite de force dans la gorge correspondante.

Selon une caractéristique particulière, les deux branches présentent chacune sur leur face interne une échancrure transversale lesdites échancrures formant, après clippage des éléments mâles dans les éléments femelles, ledit canal transversal.

Avec ce premier mode de réalisation de l'élément de ligature le coulisseau se présente sous la forme d'une gouttière ayant deux lèvres axiales parallèles, lesdites lèvres présentant en vis-à-vis à l'une de leurs extrémités une paire de bossages destinés à coulisser dans des rainures longitudinales ménagées dans la face externe d'au moins l'une des deux branches dudit élément de ligature et dans la face externe de la paroi latérale du logement, les faces externes desdites branches présentant dans lesdites rainures au moins une paire de protubérances qui par coopération avec la paire de bossages dudit coulisseau, lors d'un déplacement axial de ce dernier, entraînent le pincement des deux branches dudit élément de ligature et le clippage des moyens de fixation.

Afin d'assurer positivement l'écartement des deux branches de l'élément de ligature, lesdites deux branches se prolongent au-delà de la charnière, et la rainure de la deuxième branche présente dans le prolongement correspondant une deuxième protubérance qui par coopération avec un bossage du coulisseau, lors d'un déplacement axial de ce dernier, provoque l'écartement de la deuxième branche par rapport à la première branche.

Selon une autre caractéristique avantageuse, le bord de la gouttière, qui s'étend entre la paire de bossages, est disposé sensiblement dans un plan oblique et est biseauté afin de servir d'outil pour couper la structure anatomique enserrée par ledit élément de ligature, par déplacement axial du coulisseau.

Selon un deuxième mode de réalisation de l'élément de ligature les deux branches de l'élément de ligature sont élastiquement déformables, la première branche présente, sur sa face interne au voisinage de son extrémité proximale un passant présentant une extrémité proximale et une extrémité distale, et dans l'orifice duquel la partie proximale de la deuxième branche, introduite par l'extrémité distale est susceptible de coulisser , et la deuxième branche comporte à sa partie proximale des moyens de blocage coopérant avec ledit passant.

Le logement comporte alors en outre un ressort à lame raccordé à l'extrémité distale de la paroi latérale dudit logement par un arceau flexible, ledit ressort à lame présentant en regard de ladite paroi latérale une surface bombée en appui contre la face externe de la deuxième branche dudit élément de ligature, et le coulisseau est constitué d'une bague traversée par ladite paroi latérale et ledit ressort à lame le déplacement axial de ladite bague le long de ladite paroi latérale entraînant le rapprochement ou l'écartement dudit ressort à lame de ladite paroi latérale.

Selon une variante du deuxième mode de réalisation de l'élément de ligature, le passant présente, dans sa paroi distante de la face interne de la première branche, une fente longitudinale ouverte à ses deux extrémités, et la deuxième branche présente dans le prolongement de son extrémité proximale une languette susceptible d'être introduite dans ledit passant par coulissement à travers ladite fente ladite languette ayant une longueur supérieure à la longueur du passant et comportant à son extrémité libre un moyen de préhension.

L'applicateur comporte alors un réceptacle pour accueillir ledit moyen de préhension , ledit réceptacle étant axialement mobile et relié à un deuxième organe de manoeuvre prévu à l'extrémité proximale du corps par une deuxième tige montée coulissante dans ledit corps.

D'autres avantages et caractéristiques de l'invention ressortiront à la lecture de la description suivante faite à titre d'exemple et en référence aux dessins annexés dans lesquels :
la figure 1 montre en perspective un élément de ligature selon un premier mode de réalisation de l'invention ;
la figure 2 montre une deuxième variante de l'élément de ligature selon le premier mode de réalisation de l'invention ;
la figure 3 montre une forme différente des éléments de clippage mâles et femelles ;
la figure 4 est une vue de dessus de l'élément de ligature dans une position non verrouillée ;
la figure 5 est une vue de dessus de l'élément de ligature de la figure 4 en position verrouillée ;
les figures 6 et 7 sont des vues de la face interne d'une branche de l'élément de ligature qui montrent des variantes de réalisation des échancrures ;
la figure 8 montre une troisième variante de l'élément de ligature selon le premier mode de réalisation de l'invention ;
la figure 9 est une vue de dessus de l'élément de ligature de la figure 8 en position verrouillée ;
la figure 10 montre une quatrième variante simplifiée de l'élément de ligature selon le premier mode de réalisation selon l'invention ;
la figure 11 montre un applicateur particulièrement adapté pour mettre en place l'élément de ligature montré sur les figures 1 et 2 ;
la figure 12 montre en perspective l'extrémité distale de l'applicateur de la figure 11 sans le coulisseau ;
la figure 13 est une coupe selon un plan médian transversal de l'extrémité distale de l'applicateur sans le coulisseau ;
la figure 14 est une coupe selon un plan perpendiculaire à l'axe de l'applicateur de l'extrémité distale de ce dernier avec le coulisseau ;
la figure 15 est une vue en perspective du coulisseau ;
la figure 16 montre une variante du premier mode de réalisation de l'applicateur ;
la figure 17 montre une variante de réalisation de la rainure de la paroi latérale du logement de l'élément de ligature ;
la figure 18 montre en perspective un deuxième mode de réalisation de l'élément de ligature selon l'invention ;
la figure 19 montre une variante du deuxième mode de réalisation de l'élément de ligature selon l'invention ;
la figure 20 montre une manière possible de ligaturer une structure anatomique par l'élément de ligature souple montré sur la figure 19, sans système d'application, en utilisant une instrumentation coelioscopique standard;
la figure 21 montre l'extrémité distale d'un applicateur spécifique pour mettre en place l'élément de ligature montré sur la figure 18 ;
la figure 22 montre en perspective l'extrémité distale d'un applicateur spécifique pour mettre en place l'élément de ligature semblable à celui montré sur la figure 19.

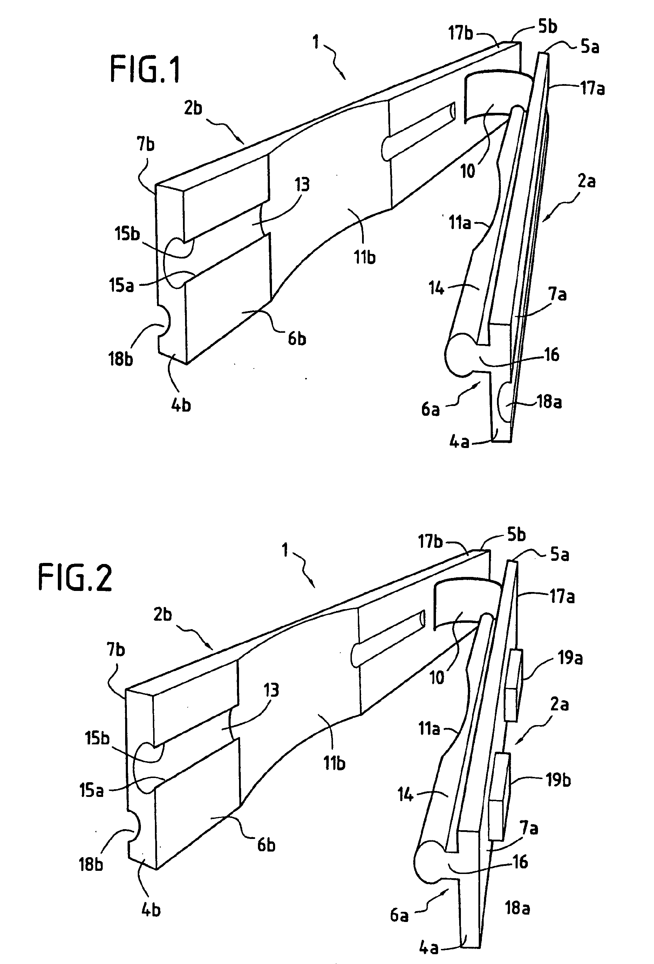

Les figures 1 à 10 montrent un élément 1 pour ligaturer notamment d'une structure anatomique oblongue, qui comportent deux branches rigides 2a, 2b présentant chacune une extrémité proximale 4a, 4b, une extrémité distale 5a, 5b, une face interne 6a, 6b et une face externe 7a, 7b, l'indice a des références 4 à 7 se rapportant à la première branche 2a, et l'indice b des références 4 à 7 se rapportant à la deuxième branche. Une bande mince flexible 10 relie les extrémités distales 5a, 5b des deux branches 2a et 2b. L'élément de ligature 1 est réalisé de préférence par moulage ou par découpe d'un matériau approprié répondant aux exigences médicales, et notamment d'un matériau résorbable, et la bande mince flexible 10 est calculée de telle manière qu'au repos, les deux branches 2a et 2b forment un angle aigu.

Ainsi que cela se voit clairement sur les figures 1 et 2, qui concernent des éléments de ligature 1 destinés à être mis en place par un applicateur décrit plus loin dans le présent mémoire, les faces internes 6a et 6b des branches 2a et 2b présentent chacune une échancrure 11a, 11b transversale qui formera un canal transversal 12 destiné à enserrer une portion de la structure anatomique.

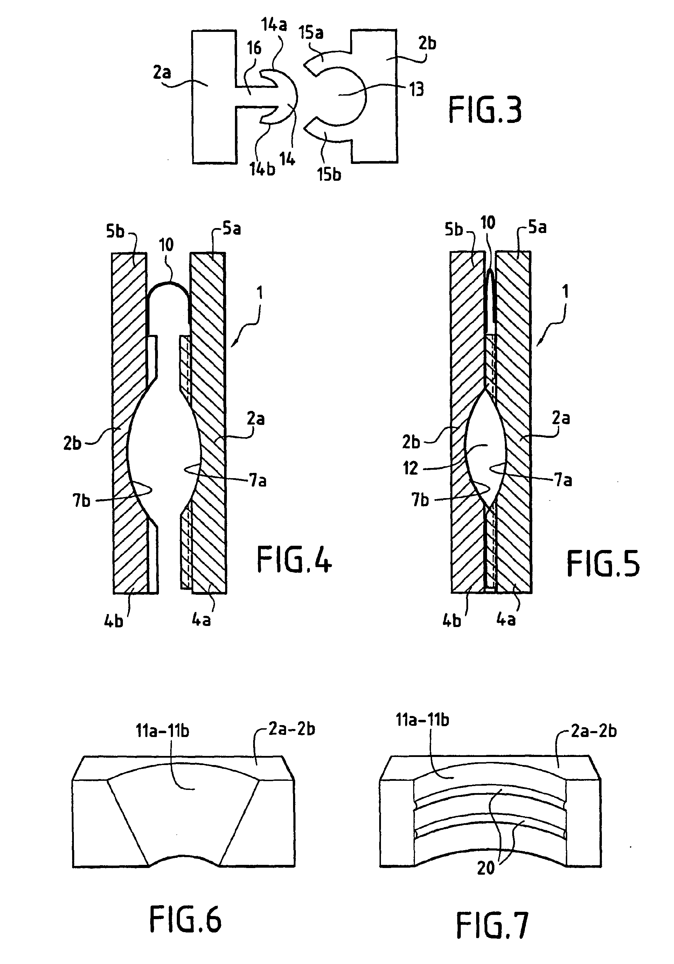

Dans la face interne 6b de la deuxième branche 2b, est ménagée une gorge longitudinale 13 destinée à recevoir de force une paroi cylindrique 14 prévue sur la face interne 6a de la première branche 2a. La gorge 13 est réalisée à contre-dépouille et elle présente sur la face interne 6b des lèvres 15a, 15b qui se déforment élastiquement lors de l'introduction de la paroi cylindrique 14, cette introduction étant obtenue en rapprochant les deux branches 2a, 2b et en exerçant sur elles des efforts de compression. La paroi cylindrique 14 est raccordée à la première branche 2a par une paroi 16 qui a une épaisseur au plus égale à la distance qui sépare les deux lèvres 15a et 15b. Le diamètre de la paroi cylindrique 14 est au plus égal au diamètre de la gorge 13. Les dimensions de la gorge 13 et de la paroi cylindrique 14 sont calculées de telle manière que la paroi cylindrique 14 puisse être introduite dans la gorge 13 par clippage, les faces internes 6a et 6b des deux branches 2a et 2b étant alors accolées, ainsi que cela est visible sur la figure 5, où l'on voit clairement le canal transversal 12 formé par les échancrures 7a et 7b.

Sur les figures 1 et 2, on voit que les branches 2a et 2b présentent à leurs extrémités distales 5a et 5b des portions d'extrémité 17a et 17b situées au-delà du raccordement de la bande mince flexible 10. Ces deux portions d'extrémité 17a et 17b sont destinées à permettre l'écartement des deux branches 2a et 2b lors de la mise en place par l'applicateur décrit plus loin afin de pouvoir crocheter la structure anatomique.

Ainsi que cela est montré sur la figure 1, les deux branches 2a et 2b présentent sur leurs faces externes 7a et 7b, des rainures longitudinales 18a, 18b, servant au guidage d'un coulisseau de l'applicateur. La rainure longitudinale 18b de la branche 2b au moins présente des protubérances non montrées sur les figures 1 et 2, qui par coopération avec un coulisseau de l'applicateur permettront de rapprocher les deux branches 2a et 2b et l'introduction de la paroi cylindrique 14 dans la gorge 13. Ces protubérances sont formées notamment près de l'extrémité proximale 4a, et éventuellement à d'autres endroits. La portion d'extrémité 17b de la branche 2b présente également dans la rainure 18b une protubérance pour écarter la branche 2b de la branche 2a.

La figure 2 montre sur la face externe 7a de la branche 2a des protubérances 19a servant au maintien de l'élément de ligature 1 sur l'applicateur, ces protubérances coopérant également avec le coulisseau pour comprimer les deux branches 2a et 2b lors de l'opération de clipsage, ainsi que cela est décrit plus en détail dans la suite du présent mémoire.

La figure 3 montre une variante de réalisation des moyens de clipsage. La gorge 13 comporte des parois élastiques se terminant par les lèvres 15a et 15b. La paroi cylindrique 14 comporte également des lèvres 14a et 14b flexibles qui se rapprochent lors de leur passage entre les lèvres 15a et 15b.

La figure 4 montre l'élément de ligature dans une position de transport vers le lieu de son application. Les deux branches 2a et 2b sont parallèles entre elles, mais non liées par les éléments de clippage 13 et 14.

Dans cette position, l'élément de ligature 1 présente une section relativement faible.

La figure 5 montre l'élément de ligature 1 après fixation des deux branches 2a et 2b entre elles. La section du canal transversal 12 est adaptée à la section de la structure anatomique sur laquelle l'élément de ligature 1 est implanté.

Les figures 6 et 7 montrent deux variantes de réalisation des échancrures 11a et 11b. La figure 6 montre une échancrure ayant une forme évasée vers le haut, tandis que la figure 6 montre une échancrure ayant des génératrices parallèles mais comportant des demi-joncs 20 dans des plans transversaux qui permettent de bien enserrer la structure anatomique.

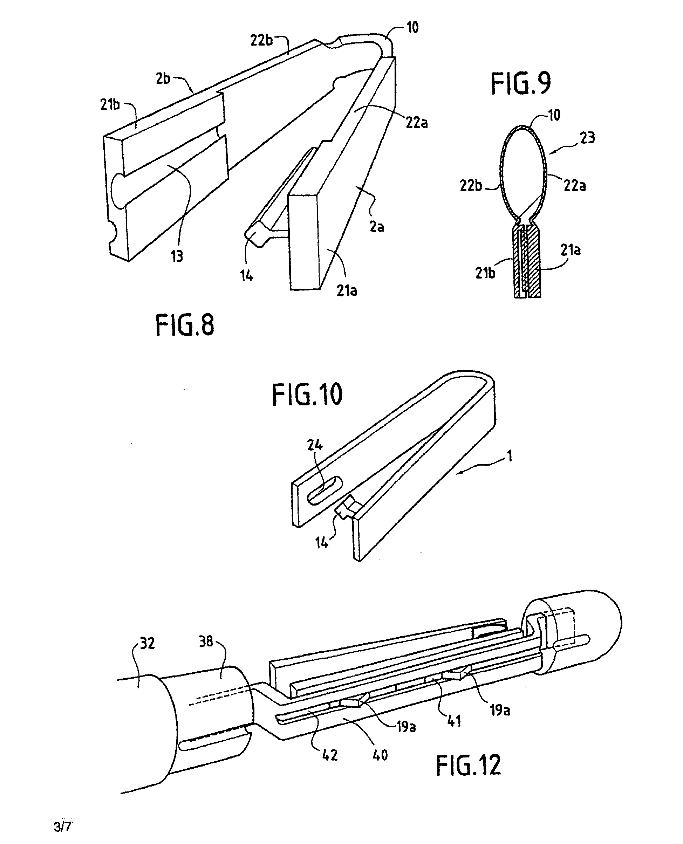

Les figures 8 et 9 montrent un élément de ligature 1 dans lequel seules les portions proximales 21a et 21b des branches 2a et 2b comportent des moyens de clippage 13 et 14, tels que ceux décrits ci-dessus, les autres portions 22a, 22b des branches 2a et 2b étant plus minces et susceptibles de se déformer élastiquement pour former, avec la charnière 10, une boucle 23, après clippage ainsi que cela est représenté sur la figure 9.

La figure 10 montre une variante simplifiée de l'élément de ligature 1, dans lequel la gorge 13 est remplacée par une fenêtre 24 à travers laquelle vient se bloquer l'élément de clippage mâle 14.

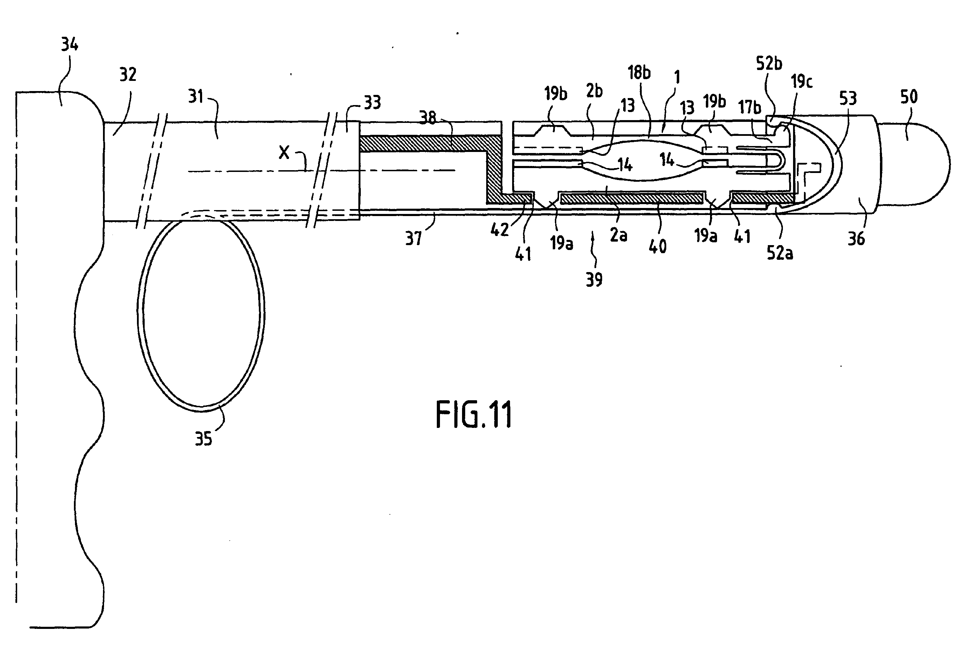

Les figures 11 à 17 montrent en détail un applicateur 30 pour mettre en place l'élément de ligature 1, montré sur les figures. 1 et 2.

L'applicateur 30 comporte un tube cylindrique 31 qui présente une extrémité proximale 32 et une extrémité distale 33. Le corps 31 a un petit diamètre, par exemple 5 mm, et est destiné à passer par une canule disposée, au moyen d'un trocart à travers la paroi d'un patient

L'extrémité proximale 32 du corps 31 est de préférence équipée d'une poignée de préhension 34. En regard de la poignée de préhension, le corps 31 est équipé d'un organe de manoeuvre 35 monté axialement mobile sur le corps 31 et relié à un coulisseau 36, décrit plus en détail plus loin dans le présent mémoire, par une tige 37 montée coulissante dans le corps 31.

Le corps 31 présente à son extrémité distale 33, une portion 38, de diamètre rétréci, susceptible de recevoir le coulisseau 36. Cette portion rétrécie 38 est prolongée par un logement 39 destiné à accueillir un élément de ligature 1. Le logement 39 est constitué essentiellement par une plaque en forme de U qui comporte une paroi latérale ou de fond 40 parallèle à l'axe X du corps 31 et contre laquelle la face externe de la première branche 2a de l'élément de ligature 1 est retenue au moyen des protubérances 19a logeant dans des fenêtres 41 ménagées dans la paroi latérale 40, ces fenêtres débouchant elles-mêmes dans une rainure longitudinale 42 ménagée sur la face externe de la paroi latérale 40. Les protubérances 19a et 19b émergent dans la rainure 42.

Ainsi que cela est montré sur la figure 11, la face externe de la deuxième branche 2a présente également dans la rainure 18b, deux protubérances 19 opposées aux deux protubérances 19a, chaque paire de protubérance 19a, 19b étant positionnée axialement ou longitudinalement au niveau d'une paire de moyens de clippage 13 et 14.

On voit également sur cette figure que la rainure 18b présente à l'extrémité libre du prolongement 17b une troisième protubérance 19c.

La référence 50 désigne un embout mousse disposé à l'extrémité avant du logement 39.

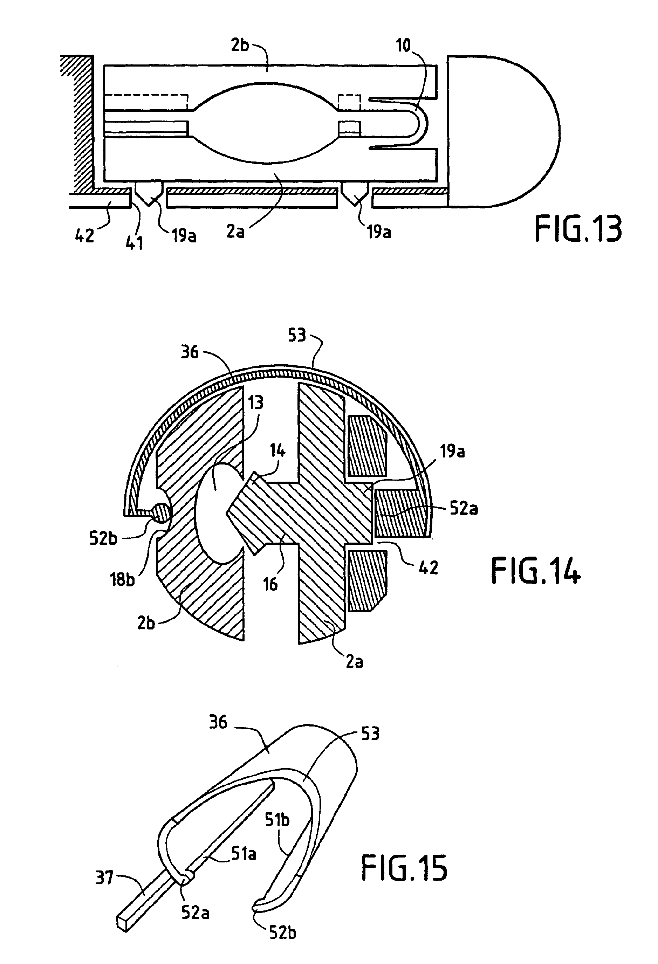

Le coulisseau 36 montré en détail sur la figure 15 se présente sous la forme d'une gouttière semi-cylindrique qui présente deux lèvres longitudinales 51a, 51b. L'une des extrémités de ces lèvres présente un bossage 52a, 52b, les deux bossages 52a et 52b étant en vis-à-vis et coulissant respectivement dans la rainure 18b de l'élément de ligature 1 et dans la rainure 42 de la paroi latérale 40 du logement 39.

La portion rétrécie 38 et l'embout de mousse 50 comportent également des rainures dans le prolongement des rainures 18b et 42.

Comme on le voit en outre sur les figures 11 et 15, le bord 53 du coulisseau qui s'étend entre les deux bossages 52a et 52b est disposé dans un plan oblique et est biseauté afin de former un outil de découpe de la structure anatomique après la ligature de cette dernière.

L'applicateur 30 décrit ci-dessus est utilisé de la manière qui va être décrite ci-après.

L'élément de ligature 1 est disposé dans le logement 39, les protubérances 19a et 19b étant positionnées dans les fenêtres 41 et le coulisseau 36 étant au niveau de l'embout mousse 50.

En déplaçant légèrement le coulisseau vers l'extrémité distale 32 du corps 31, le bossage 52b passe au-dessus de la protubérance 19c et vient se positionner entre la protubérance médiane 19b et la protubérance 19c. L'élément de ligature 1 est alors emprisonné dans le logement 39, dans la position de transport, et l'applicateur 30 est prêt à l'emploi.

Lorsqu'on a introduit l'applicateur 30 par une canule dans le corps du patient, on doit ouvrir l'élément de ligature 1 afin de crocheter la structure anatomique. Pour ce faire, l'opérateur déplace manuellement l'organe de manoeuvre 35 vers l'extrémité distale 33. Le coulisseau se déplace axialement, et le bossage 52b appuie sur la protubérance 19c du prolongement 17b de la deuxième branche 2b, ce qui écarte la deuxième branche 2b de la première branche 2a toujours maintenue contre la paroi latérale 40.

Dès qu'on a crocheté la structure anatomique, l'opérateur tire manuellement sur l'organe de manoeuvre 35, le coulisseau 36 se déplace axialement dans la direction de l'extrémité proximale 32, et les bossages 52a et 52b rencontrent les premières paires de protubérances 19a, 19b, ce qui rapproche les portions distales des branches 2a, 2b et entraîne le clippage d'une paroi cylindrique 14 dans la gorge adjacente.

En poursuivant la traction de l'organe de manoeuvre 35, les bossages 52a et 52b viennent au contact des protubérances 19a et 19b situées au voisinage des extrémités proximales 4a, 4b des deux branches 2a et 2b, et assurent le clippage des portions proximales des branches 2a et 2b lors du franchissement de ces protubérances. L' élément de ligature 1 est alors en place. En poursuivant son déplacement vers l'extrémité proximale 32, le bord biseauté 53 du coulisseau 36 coupe la structure anatomique au-dessus de la ligature réalisée.

Lorsque le coulisseau 36 sera positionné sur la portion rétrécie 38 du corps cylindrique 31, l'élément de ligature 1 est libéré, et ce d'autant plus facilement que le bossage 52a aura dégagé en partie les protubérances 19a et 19b des fenêtres 41 lors du déplacement axial du coulisseau 36.

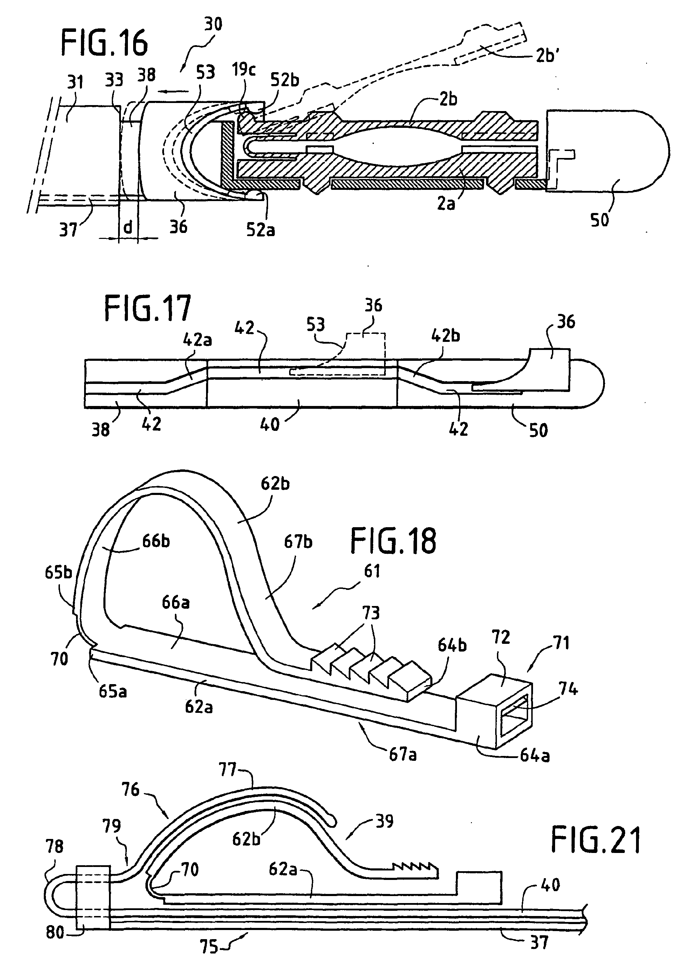

La figure 16 montre une variante de réalisation de l'applicateur 30 décrit ci-dessus.

Dans cette variante, les extrémités proximales 4a, 4b des deux branches 2a, 2b de l'élément de ligature 1 sont disposées au voisinage de l'embout mousse 50, et le coulisseau 36 présente les bossages 52a, 52b et le bord coupant 53 du côté de l'embout mousse 50.

Sur la figure 16, on a montré en trait plein la position de l'élément de ligature 1 et du coulisseau 36 lorsque l'applicateur 30 est prêt à être utilisé. Le bossage 52b se trouve à l'avant de la protubérance 19c. Le coulisseau 36 est au niveau de la portion rétrécie 38, sa face arrière étant à une distance d de l'extrémité distale du corps 31. Si l'opérateur recule le coulisseau 36 de la distance d, le bossage 52b vient appuyer sur la protubérance 19c. La branche 2b s'écarte de la branche 2a et prend la position montrée en pointillé et référencée 2b'. Au lieu de former un crochet, l'élément de ligature 1 se présente sous la forme d'une pince que l'opérateur peut positionner sur la structure anatomique. Par déplacement du coulisseau 36 vers l'embout mousse 50, on provoque successivement la fermeture et le clippage des deux branches 2a et 2b, puis la découpe de la structure anatomique enserrée dans le canal transversal 12 de l'élément de ligature 1.

Il est à noter que lorsque l'élément de ligature 1 est disposé dans le logement 39 en position de transport, c'est-à-dire les deux branches 2a et 2b étant parallèles entre elles, mais non verrouillées, la section transversale de l'ensemble constitué par le logement 39, l'élément de ligature 1 et le coulisseau 36 est au plus égale à celle du corps cylindrique 31 afin de passer par la canule du trocart.

Il est à noter, en outre, que la rainure 42 ménagée sur la face externe de la paroi latérale 40 du logement 39 sur la portion rétrécie 38 et sur l'embout mousse 50 peut présenter des tronçons 42a, 42b obliques, afin d'éloigner le bord tranchant 53 de l'élément de ligature 1 lors de la coupe de la structure anatomique, ainsi que cela est montré sur la figure 17.

La figure 18 montre un élément de ligature 61 selon un deuxième mode de réalisation qui comporte une première branche 62a semi-rigide et une deuxième branche 62b également semi-rigide présentant chacune une extrémité proximale 64a, 64b, une extrémité distale 65a, 65b, une face interne 66a, 66b, et une face externe 67a, 67b. Une bande mince flexible 70 relie les extrémités distales 65a, 65b des deux branches 62a et 62b. Cet élément de ligature 61 est également réalisé, de préférence, par moulage d'un matériau approprié, répondant aux exigences médicales, en une ou plusieurs pièces assemblables.

La première branche 62a présente sur sa face interne 66a au voisinage immédiat de son extrémité proximale 64a un passant 71, de section sensiblement rectangulaire, ayant une paroi 72 distante de la face interne 66a.

La deuxième branche 62b présente sur la face externe 67b de sa portion proximale une pluralité de crans 73 transversaux. La section du conduit 74 du passant 72 est calculée de telle manière que la portion proximale de la deuxième branche, introduite par l'extrémité distale du conduit 74, puisse coulisser de force ou à légère friction dans le conduit 74, l'un des crans 73 venant ensuite en appui sur l'extrémité proximale de la paroi 72 pour empêcher un retour arrière de la portion proximale, ce qui entraîne la fixation des portions proximales des deux branches 62a et 62b entre elles.

Les crans 73 peuvent être disposés latéralement sur la portion proximale de la deuxième branche 62b ou être remplacés par de simples aspérités, picots ou rugosités, qui entraînent des forces de frottement assurant le serrage.

La figure 21 montre l'extrémité distale 75 d'un applicateur 30 pour mettre en place l'élément de ligature 61 selon le deuxième mode de réalisation. Cet applicateur 30 est sensiblement semblable à celui décrit plus haut et servant à mettre en place l'élément de ligature 1 selon le premier mode de réalisation de l'invention. Les seules différences concernent le logement 39 de l'élément de ligature 61 et le coulisseau 36 relié à l'organe de manoeuvre 35 par une tige 37 mobile axialement.

Le logement 39 comporte une paroi latérale 40, et un ressort à lame 76 qui présente en regard de la paroi latérale 40 une surface 77 bombée reliée à l'extrémité distale 78 de la paroi latérale 40 par un arceau 79. Le coulisseau 36 est constitué d'une bague 80 fixée à l'extrémité distale de la tige 37. La bague 80 est disposée dans un plan perpendiculaire à l'axe X de l'applicateur 30, et elle est traversée par la paroi latérale 40 et le ressort à lame 76.

Lors du déplacement axial du coulisseau 76, le ressort à lame 76 et la paroi latérale 40 coulissent dans l'alésage de la bague 80.

La surface bombée 77 se raccorde à l'arceau 73 par une zone de raccordement 79 à courbure inversée.

L'élément de ligature 61 est disposé entre la paroi latérale 40 et le ressort à lame 76. La première branche 62a est en appui contre la paroi latérale 40 et la deuxième branche 62b est en appui contre la surface bombée 77. La bande flexible mince 70 est disposée en regard de l'arceau 78.

Lorsque la bague 80 est dans sa position la plus distale, la deuxième branche 62b est écartée au maximum de la première branche 62a et il est possible de crocheter une structure anatomique à ligaturer.

Lorsque l'opérateur tire sur l'organe de manoeuvre 35, la surface bombée 77 se rapproche de la paroi latérale 40 et la portion d'extrémité proximale de la deuxième branche 62b vient se mettre en regard de l'orifice 74 du passant 71. L'élément de ligature 1 est alors dans la position de transport et l'applicateur 30 peut être introduit dans la canule. La section du logement 39 est alors au plus égale à la section du corps cylindrique 31.

Pour ouvrir l'élément de ligature, l'opérateur repousse l'organe de manoeuvre 35, puis, après avoir crocheté la structure anatomique, il tire sur l'organe de manoeuvre, afin de rapprocher la deuxième branche 62b de la première branche 62a, comme représenté sur la figure 21. En tirant davantage sur l'organe de manoeuvre 35, la portion crantée de la deuxième branche 62b pénètre et coulisse dans l'orifice 74 du passant 71, ce qui enserre la structure anatomique dans le canal transversal de l'élément de ligature 61.

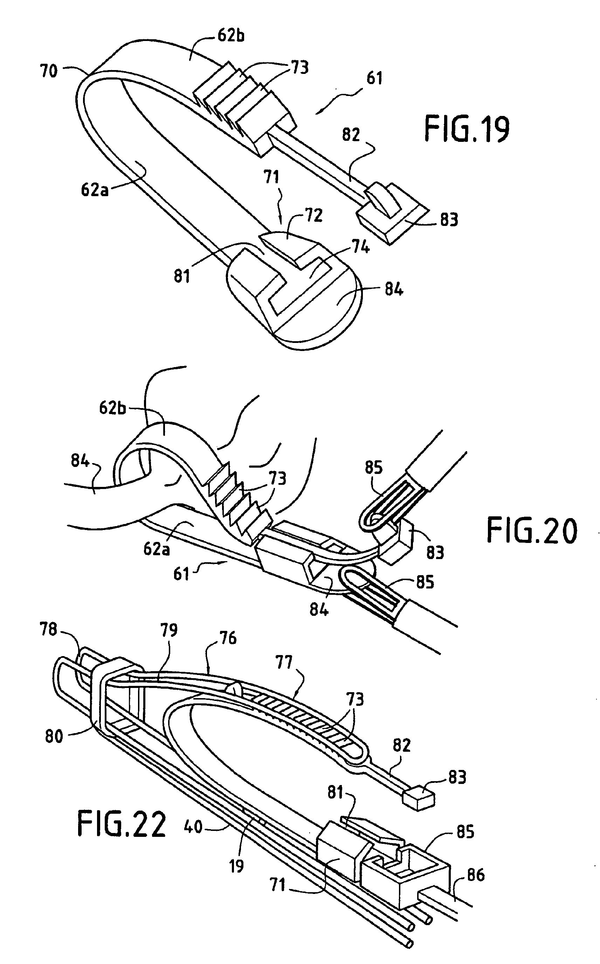

La figure 19 montre une variante de l'élément de ligature 61 selon le deuxième mode de réalisation. Cette variante diffère de celle montrée sur la figure 18, par une fente longitudinale 81 ménagée dans la paroi 72 du passant 71 et par une languette 82 située dans le prolongement de la portion crantée de la deuxième branche 62b. Cette languette 82 a une longueur légèrement supérieure à la longueur du passant 71 et peut être introduite dans le conduit 74 par coulissement à travers la fente longitudinale 81. La languette 82 se termine par un moyen de préhension 83. L'extrémité proximale de la première branche 62a peut également comporter un moyen de préhension 84. La deuxième branche 62b est dans ce cas souple. L'ensemble 61 est ,dans ce cas, constitué en totalité en matériau souple pour se comporter comme une ligature.

La figure 20 montre une manière de monter l'élément de ligature 61 décrit ci-dessus autour de la structure anatomique en prenant les languettes 84 et 85 au moyen de pinces coelioscopiques.

Cet élément de ligature 61 peut être mis en place au moyen de l'applicateur 30 décrit plus haut. Lorsque par déplacement axial de la bague 80, la surface bombée 77 se rapproche de la paroi latérale 40, la languette 82 passe d'abord par la fente 81, puis la portion crantée coulisse dans le conduit 74 du passant 71.

La figure 22 montre une variante de réalisation de l'applicateur 30. Cet applicateur 30 présente un réceptacle 85 au voisinage du logement 39 dans lequel le moyen de préhension 83 situé à l'extrémité de la languette vient se loger lorsque la longuette 82 est disposée dans le conduit 74 du passant 71. Ce réceptacle 85 est relié par une deuxième tige 86 à un deuxième organe de manoeuvre, non montré sur les dessins, situé à l'extrémité proximale du corps cylindrique 31 de l'applicateur 30.

Comme on le voit sur la figure 22, la paroi latérale 40 et le ressort à lame 76 peuvent être réalisés au moyen de fils à ressorts de section cylindrique ou aplatie. Ce ressort a un rôle important : il permet de manoeuvrer la branche 62b à laquelle il est solidarisé par le ou les ergots ; sa forme en 78 crée plusieurs plateaux successifs, la bague 80 étant poussé à l'extrémité de l'instrument , la partie 76 préformée ,se relève entraînant avec elle la branche 62b ; lorsque la bague 80 est tirée sur le cran 79 la partie 76 amène la branche 62b au contact du passant 71 et du réceptacle 85.Cette position est réputée être la position de transport ou d'introduction dans un trocart d'accès, en sachant que la boucle formée par les 2 branches 62 a et 62b sera aplatie au passage dans le trocart et que la partie effilée 82, d'une longueur calculée pour ce faire, coulissera librement dans la fente 81,et dans le réceptacle 85 dont la paroi proximale 85 bis sera suffisamment éloignée pour permettre les déplacements des parties 82 et 83. La partie rectiligne 40 du ressort contre laquelle coulisse l'axe 75 , reçoit les ergots. 19 qui maintiennent dans le logement la branche 62a et la partie postérieure du passant 71.Ce dernier vient en appui sur un relief 87 du manche de l'applicateur de telle sorte que lors de la traction du moyen de préhension 83 par la partie échancrée du réceptacles 85 , le passant 71 puisse rester fixe tandis que la branche 62b et les crans 73 sont tirés à travers la partie interne du passant 71. Lorsque la boucle formée par les branches 62a et 62b est suffisamment refermée sur la structure anatomique à contrôler, l'axe 86 est repoussé vers la partie distale de l'instrument pour dégager l'élément 61 de son applicateur.

De préférence, l'élément de ligature 1 ou 61 est pré monté en atelier sur l'applicateur 30,en engageant les ergots 19 dans des espaces ,prévus à cet effet, dans la paroi du ressort 40 et 76, pour constituer un ensemble stérile prêt à l'emploi.

Plusieurs parties de l'applicateur 30 et notamment le corps cylindrique 31 sont réalisés en un matériau plastique.

La mise en place de l'élément de ligature 1 au moyen de l'applicateur 30 se fait par l'opérateur sous le contrôle optique d'une vidéo, ce qui permet d'économiser une pince et un trocart supplémentaire, par rapport à la technique actuelle.

## Revendications

1. Dispositif pour ligaturer une structure anatomique oblongue, telle qu' un appendice iléo-cæcal ou un méso-appendice, ou toutes autres structures anatomiques vasculaires ou tissulaires, pouvant être utilisé sous coelioscopie, **caractérisé par le fait qu'**il comprend :
d'une part, un élément de ligature comportant une première branche (2a, 62a) et une deuxième branche (2b, 62b) présentant chacune une extrémité proximale (4a, 4b, 64a, 64b), une extrémité distale (5a, 5b, 65a, 65b), une face interne (6a, 6b, 66a, 66b) et une face externe (7a, 7b, 67a, 67b), une bande mince flexible (10, 70) formant charnière et reliant les extrémités distales desdites deux branches (2a, 2b) et des moyens pour lier entre elles au moins les portions proximales desdites deux branches (2a, 2b), lesdites deux branches ménageant entre elles, après leur liaison, un canal transversal (12) enserrant une portion de ladite structure anatomique, et
d'autre part, un applicateur pour mettre en place ledit élément de ligature, ledit applicateur comportant :
- un corps cylindrique (31) ayant une extrémité distale (33) et une extrémité proximale (32),
- un logement (39) pour recevoir ledit élément de ligature (1), ce logement présentant une paroi latérale (40) prévue à l'extrémité distale dudit corps (31) et contre laquelle la face externe (7a) de la première branche (2a) dudit élément de ligature est en appui notamment lors de son transport,
- des moyens (52a, 19c, 76) pour écarter la deuxième branche (2b) de la première branche (2a) afin de permettre le crochetage de la structure anatomique à ligaturer,
- des moyens (52a, 52b, 19a, 19b, 76) pour rapprocher la deuxième branche (2b) de la première branche (2a) afin de lier entre elles lesdites deux branches,
**caractérisé en ce qu'**il comporte en outre un coulisseau (36, 80) permettant d'agir sur les moyens pour écarter ou rapprocher la deuxième branche de la première branche.

2. Dispositif selon la revendication 1 **caractérisé par le fait que** le coulisseau (36) est monté mobile au moins le long de la paroi latérale (40) dudit logement (39), ledit coulisseau comportant les moyens pour écarter ou rapprocher la deuxième branche (2b) de la première branche (2a), ledit coulisseau étant susceptible d'être déplacé axialement par un organe de manoeuvre (35) disposé à l'extrémité proximale dudit corps (31) et relié audit coulisseau (36) par une tige (37) montée coulissante dans ledit corps (31).

3. Dispositif selon la revendication 2, **caractérisé par le fait que** les deux branches de l'élément de ligature sont rectilignes et rigides et les moyens pour lier entre elles lesdites deux branches (2a, 2b) comportent des éléments de clippage mâles et des éléments de clippage femelles prévus sur les faces internes (6a, 6b) desdites deux branches (2a, 2b).

4. Dispositif selon la revendication 3, **caractérisé par le fait que** les éléments de clippage femelles comportent au moins une gorge longitudinale (13) à contre-dépouille débouchant dans la face interne (6a) de l'une des branches, et les éléments de clippage mâles comportent au moins une paroi cylindrique (14) formée sur la face interne (6b) de l'autre branche et susceptible d'être introduite de force dans la gorge (13) correspondante.

5. Dispositif selon l'une des revendications 3 ou 4, **caractérisé par le fait que** les deux branches (2a, 2b) présentent chacune sur leur face interne une échancrure transversale (11a, 11b), lesdites échancrures formant, après clippage des éléments mâles dans les éléments femelles, ledit canal transversal (12).

6. Dispositif selon l'une des revendications 3 à 5, **caractérisé par le fait que** le coulisseau (36) se présente sous la forme d'une gouttière ayant deux lèvres (51a, 51b) axiales parallèles, lesdites lèvres (51a, 51b) présentant en vis-à-vis à l'une de leurs extrémités une paire de bossages (52a, 52b) destinés à coulisser dans des rainures longitudinales (18a, 18b, 42) ménagées dans la face externe d'au moins l'une des deux branches (2a, 2b) dudit élément de ligature et dans la face externe de la paroi latérale (40) du logement (39), les faces externes desdites branches (2a, 2b) présentant dans lesdites rainures au moins une paire de protubérances (19a, 19b) qui par coopération avec la paire de bossages (52a, 52b) dudit coulisseau (38), lors d'un déplacement axial de ce dernier, entraînent le pincement des deux branches (2a, 2b) dudit élément de ligature et le clippage des moyens de fixation (13, 14).

7. dispositif selon la revendication 6, **caractérisé par le fait que** les deux branches (2a, 2b) de l'élément de ligature (1) se prolongent au-delà de la charnière (10), et la rainure (18b) de la deuxième branche (2b) présente dans le prolongement (17b) correspondant une deuxième protubérance (19c) qui par coopération avec un bossage (52b) du coulisseau (36), lors d'un déplacement axial de ce dernier, provoque l'écartement de la deuxième branche (2b) par rapport à la première branche (2a).

8. Dispositif selon l'une des revendications 6 ou 7, **caractérisé par le fait que** le bord (53) de la gouttière, qui s'étend entre la paire de bossages (52a, 52b), est disposé sensiblement dans un plan oblique et est biseauté afin de servir d'outil pour couper la structure anatomique enserrée par ledit élément de ligature (1), par déplacement axial du coulisseau (36).

9. Dispositif selon la revendication 2, **caractérisé par le fait que** les deux branches (62a, 62b) de l'élément de ligature sont élastiquement déformables, et/ou semi-rigides, **par le fait que** la première branche (62a) présente, sur sa face interne (66a) au voisinage de son extrémité proximale (64a), un passant (71) présentant une extrémité proximale et une extrémité distale, et dans l'orifice (74) duquel la partie proximale de la deuxième branche (62b), introduite par l'extrémité distale est susceptible de coulisser , et la deuxième branche (62b) comporte à sa partie proximale des moyens de blocage coopérant avec ledit passant (71).

10. Dispositif selon la revendication 9, **caractérisé par le fait que** le logement (39) comporte en outre un ressort à lame (76) raccordé à l'extrémité distale de la paroi latérale (40) dudit logement (39) par un arceau flexible (78), ledit ressort à lame (76) présentant en regard de ladite paroi latérale (40) une surface bombée (77) en appui contre la face externe (67b) de la deuxième branche (62b) dudit élément de ligature, et le coulisseau (36) est constitué d'une bague (80) traversée par ladite paroi latérale (40) et ledit ressort à lame (76), le déplacement axial de ladite bague (80) le long de ladite paroi latérale (40) entraînant le rapprochement ou l'écartement dudit ressort à lame (76) de ladite paroi latérale (40).

11. Dispositif selon la revendication 10, **caractérisé par le fait que** le passant (71) présente, dans sa paroi (72) distante de la face interne (66a) de la première branche (62a), une fente longitudinale (81) ouverte à ses deux extrémités, et la deuxième branche (62b) présente dans le prolongement de son extrémité proximale une languette (82) susceptible d'être introduite dans ledit passant (71) par coulissement à travers ladite fente (81), ladite languette (82) ayant une longueur supérieure à la longueur du passant et comportant à son extrémité libre un moyen de préhension (83).

12. Dispositif selon la revendication 11 **caractérisé par le fait que** l'élément de ligature est constitué de matériau souple permettant d'engager des crans (73) disposés sur la deuxième branche (62b) dans le passant (71), la traction sur le moyen de préhension (83) rapprochant les première et deuxième branches de façon progressive pour enserrer et maintenir la structure anatomique (84).

13. Dispositif selon la revendication 11 **caractérisé par le fait que** ledit applicateur comporte un réceptacle (85) pour accueillir ledit moyen de préhension (83), ledit réceptacle (85) étant axialement mobile et relié à un deuxième organe de manoeuvre prévu à l'extrémité proximale du corps par une deuxième tige (86) montée coulissante dans ledit corps (31).

## Claims

1. A device for ligating an oblong anatomical structure such as a vermiform appendix or a mesoappendix, or any other vessel or tissue anatomic structures, the device being suitable for being used under celioscopy, and being **characterized by** the fact that it comprises:
firstly, a ligature element having a first branch (2a, 62a) and a second branch (2b, 62b), each of which has a proximal end (4a, 4b, 64a, 64b), a distal end (5a, 5b, 65a, 65b), an inside face (6a, 6b, 66a, 66b) and an outside face (7a, 7b, 67a, 67b), a flexible thin strip (10, 70) forming a hinge and interconnecting the distal ends of said two branches (2a, 2b), and means for coupling together at least the proximal portions of said two branches (2a, 2b), said two branches co-operating, once they have been coupled together, to form between them a transverse channel (12) clamping around a portion of said anatomical structure; and
secondly, an applicator for putting in place said ligature element, said applicator comprising:
- a cylindrical body (31) having a distal end (33) and a proximal end (32);
- a holder (39) for receiving said ligature element (1), said holder having a side wall (40) provided at the distal end of said body (31) and against which the outside face (7a) of the first branch (2a) of said ligature element is in abutment, in particular while it is being transported;
- means (52a, 19c, 76) for moving the second branch (2b) away from the first branch (2a) for the purpose of hooking onto the anatomical structure to be ligated;
- means (52a, 52b, 19a, 19b, 76) for moving the second branch (2b) towards the first branch (2a) in order to couple said two branches together;
the device being **characterized in that** it further comprises a slide (36, 80) making it possible to act on the means for moving the second branch away from or towards the first branch.

2. A device according to claim 1, **characterized by** the fact that the slide (36) is mounted to move at least along the side wall (40) of said holder (39), said slide including the means for moving the second branch (2b) away from or towards the first branch (2a), said slide being suitable for being moved axially by a drive member (35) disposed at the proximal end of said body (31) and connected to said slide (36) by a rod (37) mounted to slide in said body (31).

3. A device according to claim 2, **characterized by** the fact that the two branches of the ligature element are rectilinear and rigid, and the means for coupling the two branches (2a, 2b) together comprise male snap-fastening elements and female snap-fastening elements provided on the inside faces (6a, 6b) of said two branches (2a, 2b).

4. A device according to claim 3, **characterized by** the fact that the female snap-fastening elements comprise at least one longitudinal groove (13) having reentrant edges and opening out in the inside face (6a) of one of the branches, and the male snap-fastening elements comprise at least one cylindrical wall (14) formed on the inside face (6b) of the other branch and suitable for being inserted by force into the corresponding groove (13).

5. A device according to claim 3 or claim 4, **characterized by** the fact that the inside face of each of the two branches (2a, 2b) is provided with a transverse recess (11a, 11b), said recesses co-operating, after the male elements have been snap-fastened into the female elements, to form said transverse channel (12).

6. A device according to any one of claims 3 to 5, **characterized by** the fact that the slide (36) is in the form of a trough having two parallel axial lips (51a, 51b), said lips (51a, 51b) having, at one end, a pair of facing projections (52a, 52b) designed to slide in longitudinal guide groves (18a, 18b, 42) provided in the outside face of at least one of the two branches (2a, 2b) of said ligature element and in the outside face of the side wall (40) of the holder (39), the outside faces of said branches (2a, 2b) having, in said guide grooves, at least one pair of protuberances (19a, 19b) which, by co-operating with the pair of projections (52a, 52b) of said slide (38), while said slide is moving axially, cause the two branches (2a, 2b) of said ligature element to clamp together and cause the fastening means (13, 14) to snap-fasten together.

7. A device according to claim 6, **characterized by** the fact that the two branches (2a, 2b) of the ligature element (1) are extended beyond the hinge (10), and the groove (18b) of the second branch (2b) has a second protuberance (19c) in the corresponding extension (17b), which second protuberance co-operates with a projection (52b) on the slide (36) while said slide is moving axially to cause the second branch (2b) to move away from the first branch (2a).

8. A device according to claim 6 or claim 7, **characterized by** the fact that the edge (53) of the trough that extends between the pair of projections (52a, 52b) is disposed substantially in a slanting plane and is beveled in order to serve as a tool for cutting off the anatomical structure clamped by said ligature element (1), by the slide (36) moving axially.

9. A device according to claim 2, **characterized by** the fact that the two branches (62a, 62b) of the ligature element are resiliently deformable and/or semi-rigid, by the fact that, on its inside face (66a) and in the vicinity of its proximal end (64a), the first branch (62a) has a keeper (71) having a proximal end and a distal end, and, in whose orifice (74) the proximal portion of the second branch (62b) inserted via the distal end is slidably received, and, at its proximal end, the second branch (62b) has locking means co-operating with said keeper (71).

10. A device according to claim 9, **characterized by** the fact that the holder (39) is further provided with a spring blade (76) connected to the distal end of the side wall (40) of said holder (39) via a flexible hoop (78), said spring blade (76) having a convex surface (77) facing said side wall (40), which convex surface is in abutment: against the outside face (67b) of the second branch (62b) of said ligature element, and the slide (36) is constituted by a ring (80) through which said side wall (40) and said spring blade (76) pass, axial movement of said ring (80) along said side wall (40) causing said spring blade (76) to move towards or away from said side wall (40).

11. A device according to claim 10, **characterized by** the fact that, in its wall (72) distant from the inside face (6a) of the first branch (62a), the keeper (71) is provided with a longitudinal slot (81) open at both of its ends, and, extending its proximal end, the second branch (62b) has a tongue (82) suitable for being inserted into said keeper (71) by sliding through said slot (81), said tongue (82) having a length longer than the length of the keeper and having graspable means (83) at its free end.

12. A device according to claim 11, **characterized by** the fact that the ligature element is made of a flexible material making it possible to engage serrations (73) disposed on the second branch (62b) into the keeper (71), traction exerted on the graspable means (83) causing the first and the second branches to move towards each other gradually so as to clamp and hold the anatomical structure (84).

13. A device according to claim 11, **characterized by** the fact that said applicator is provided with a receptacle (85) for receiving said graspable means (83), said receptacle (85) being mounted to move axially and being connected to a second drive member provided at the proximal end of the body via a second rod (86) mounted to slide in said body (31).

## Patentansprüche

1. Vorrichtung zum Unterbinden einer länglichen anatomischen Struktur, wie eines Appendix vermiformis oder einer Mesoappendix, oder aller anderen anatomischen Gefäß- oder Gewebestrukturen, die während einer Zölioskopie verwendet werden kann, **dadurch gekennzeichnet, daß** sie umfaßt:
einerseits ein Ligaturelement, umfassend einen ersten Schenkel (2a, 62a) und einen zweiten Schenkel (2b, 62b), die jeweils ein proximales Ende (4a, 4b, 64a, 64b), ein distales Ende (5a, 5b, 65a, 65b), eine Innenseite (6a, 6b, 66a, 66b) und eine Außenseite (7a, 7b, 67a, 67b) aufweisen, ein dünnes flexibles Band (10, 70), das ein Scharnier bildet und die distalen Enden der beiden Schenkel (2a, 2b) verbindet, sowie Mittel zum Verbinden wenigstens der proximalen Abschnitte der beiden Schenkel (2a, 2b), wobei die beiden Schenkel nach ihrem Verbinden zwischen sich einen querverlaufenden Kanal (12) aussparen, der einen Abschnitt der anatomischen Struktur einspannt, sowie
andererseits einen Applikator, um das Ligaturelement einzusetzen, wobei der Applikator umfaßt:
- einen zylindrischen Körper (31), der ein distales Ende (33) und ein proximales Ende (32) aufweist,
- eine Aufnahme (39), um das Ligaturelement (1) aufzunehmen, wobei diese Aufnahme eine Seitenwand (40) aufweist, die an dem distalen Ende des Körpers (31) vorgesehen ist und an der die Außenseite (7a) des ersten Schenkels (2a) des Ligaturelements insbesondere während seines Transports in Anlage ist,
- Mittel (52a, 19c, 76), um den zweiten Schenkel (2b) von dem ersten Schenkel (2a) zu entfernen, um das Verhaken der zu unterbindenden anatomischen Struktur zu ermöglichen,
- Mittel (52a, 52b, 19a, 19b, 76), um den zweiten Schenkel (2b) dem ersten Schenkel (2a) zu nähern, um die beiden Schenkel untereinander zu verbinden,
**dadurch gekennzeichnet, daß** sie ferner einen Schieber (36, 80) umfaßt, der ermöglicht, auf die Mittel einzuwirken, um den zweiten Schenkel von dem ersten Schenkel zu entfernen oder um ihn diesem zu nähern.

2. Vorrichtung nach Anspruch 1, **dadurch gekennzeichnet, daß** der Schieber (36) wenigstens entlang der Seitenwand (40) der Aufnahme (39) beweglich angebracht ist, wobei der Schieber die Mittel umfaßt, um den zweiten Schenkel (2b) von dem ersten Schenkel (2a) zu entfernen oder um ihn diesem zu nähern, wobei der Schieber geeignet ist, durch ein Betätigungsorgan (35), das an dem proximalen Ende des Körpers (31) angeordnet ist und über eine in dem Körper (31) verschieblich angebrachte Stange (37) mit dem Schieber (36) verbunden ist, axial verschoben zu werden.

3. Vorrichtung nach Anspruch 2, **dadurch gekennzeichnet, daß** die beiden Schenkel des Ligaturelements geradlinig und starr sind und daß die Mittel zum Verbinden der beiden Schenkel (2a, 2b) untereinander Einsteckclipelemente sowie Aufnahmeclipelemente, die an den Innenseiten (6a, 6b) der beiden Schenkel (2a, 2b) vorgesehen sind, umfassen.

4. Vorrichtung nach Anspruch 3, **dadurch gekennzeichnet, daß** die Aufnahmeclipelemente wenigstens eine hinterschnittene Längsnut (13) umfassen, welche in die Innenseite (6a) von einem der Schenkel mündet, und die Einsteckclipelemente wenigstens eine zylindrische Wand (14) umfassen, die an der Innenseite (6b) des anderen Schenkels ausgebildet und geeignet ist, in die entsprechende Nut (13) hineingedrückt zu werden.

5. Vorrichtung nach einem der Ansprüche 3 oder 4, **dadurch gekennzeichnet, daß** die beiden Schenkel (2a, 2b) jeweils an ihrer Innenseite eine bogenförmige Queraussparung (11a, 11b) aufweisen, wobei die bogenförmigen Aussparungen nach dem Einclipsen der Einsteckelemente in die Aufnahmeelemente den Querkanal (12) bilden.

6. Vorrichtung nach einem der Ansprüche 3 bis 5, **dadurch gekennzeichnet, daß** der Schieber (36) in Form einer Rinne mit zwei parallelen, axialen Lippen (51a, 51 b) vorliegt, wobei die Lippen (51 a, 51 b) gegenüberliegend an einem ihrer Enden ein Paar Buckel (52a, 52b) aufweisen, die dazu bestimmt sind, in Längsnuten (18a, 18b, 42) zu gleiten, welche in der Außenseite von wenigstens einem der beiden Schenkel (2a, 2b) des Ligaturelements sowie in der Außenseite der Seitenwand (40) der Aufnahme (39) ausgebildet sind, wobei die Außenseiten der Schenkel (2a, 2b) in den Nuten wenigstens ein Paar Vorsprünge (19a, 19b) aufweisen, die durch Zusammenwirken mit dem Buckelpaar (52a, 52b) des Schiebers (38), bei einem axialen Verschieben des letzteren, das Einklemmen der beiden Schenkel (2a, 2b) des Ligaturelements und das Verclipsen der Befestigungsmittel (13, 14) bewirken.

7. Vorrichtung nach Anspruch 6, **dadurch gekennzeichnet, daß** die beiden Schenkel (2a, 2b) des Ligaturelements (1) sich jenseits des Scharniers (10) fortsetzen und die Nut (18b) des zweiten Schenkels (2b) in der entsprechenden Verlängerung (17b) einen zweiten Vorsprung (19c) aufweist, der durch Zusammenwirken mit einem Buckel (52b) des Schiebers (36), bei einem axialen Verschieben des letzteren, das Entfernen des zweiten Schenkels (2b) von dem ersten Schenkel (2a) bewirkt.

8. Vorrichtung nach einem der Ansprüche 6 oder 7, **dadurch gekennzeichnet, daß** der Rand (53) der Rinne, der sich zwischen dem Buckelpaar (52a, 52b) erstreckt, im wesentlichen in einer schrägen Ebene angeordnet und abgeschrägt ist, um als Werkzeug zum Schneiden der durch das Ligaturelement (1) eingeklemmten anatomischen Struktur durch axiales Verschieben des Schiebers (36) zu dienen.

9. Vorrichtung nach Anspruch 2, **dadurch gekennzeichnet, daß** die beiden Schenkel (62a, 62b) des Ligaturelements **dadurch** elastisch verformbar und/oder halb starr sind, daß der erste Schenkel (62a) an seiner Innenseite (66a), in der Nähe seines proximalen Endes (64a), eine Schlaufe (71) aufweist, die ein proximales Ende und ein distales Ende aufweist und in deren Öffnung (74) der proximale Teil des zweiten Schenkels (62b), mit dem distalen Ende eingeführt, zu gleiten in der Lage ist, und der zweite Schenkel (62b) an seinem proximalen Teil Blockiermittel umfaßt, die mit der Schlaufe (71) zusammenwirken.

10. Vorrichtung nach Anspruch 9, **dadurch gekennzeichnet, daß** die Aufnahme (39) ferner eine Blattfeder (76) umfaßt, die mit dem distalen Ende der Seitenwand (40) der Aufnahme (39) über einen flexiblen Bügel (78) verbunden ist, wobei die Blattfeder (76) gegenüber der Seitenwand (40) eine gewölbte Fläche (77) aufweist, die in Anlage an der Außenseite (67b) des zweiten Schenkels (62b) des Ligaturelements ist, und der Schieber (36) von einem Ring (80) gebildet ist, der von der Seitenwand (40) und der Blattfeder (76) durchgriffen ist, wobei das axiale Verschieben des Rings (80) entlang der Seitenwand (40) das Annähern oder das Entfernen der Blattfeder (76) an die bzw. von der Seitenwand (40) bewirkt.

11. Vorrichtung nach Anspruch 10, **dadurch gekennzeichnet, daß** die Schlaufe (71) in ihrer von der Innenseite (66a) des ersten Schenkels (62a) entfernten Wand (72) einen an seinen beiden Enden offenen Längsschlitz (81) aufweist und der zweite Schenkel (62b) in der Verlängerung seines proximalen Endes eine Zunge (82) aufweist, die geeignet ist, durch Schieben durch den Schlitz (81) in die Schlaufe (71) eingeführt zu werden, wobei die Zunge (82) eine größere Länge als die Schlaufe aufweist und an ihrem freien Ende ein Greifmittel (83) umfaßt.

12. Vorrichtung nach Anspruch 11, **dadurch gekennzeichnet, daß** das Ligaturelement aus einem flexiblen Material besteht, das ermöglicht, Rasten (73), die an dem zweiten Schenkel (62b) angeordnet sind, in die Schlaufe (71) einzustecken, wobei das Ziehen an dem Greifmittel (83) den ersten und den zweiten Schenkel schrittweise nähert, um die anatomische Struktur (84) festzuklemmen und zu halten.

13. Vorrichtung nach Anspruch 11, **dadurch gekennzeichnet, daß** der Applikator eine Aufnahme (85) zum Aufnehmen des Greifmittels (83) umfaßt, wobei die Aufnahme (85) axial beweglich und über eine in dem Körper (31) verschieblich angebrachte zweite Stange (86) mit einem zweiten Betätigungsorgan, das an dem proximalen Ende des Körpers vorgesehen ist, verbunden ist.
